# EUROPÄISCHE PATENTSCHRIFT

(11) **EP 1 381 335 B1**
(45) Veröffentlichungstag und Bekanntmachung des Hinweises auf die Patenterteilung: **17.06.2009**
(21) Anmeldenummer: 02737956.9
(22) Anmeldetag: 15.04.2002
(51) Int. Cl.: A61F 2/38

(54) **KNIEPROTHESE MIT EINEM BEUGESCHARNIER**
KNEE PROSTHESIS WITH A FLEXION HINGE
PROTHESE DU GENOU POURVUE D'UNE CHARNIERE DE FLEXION

(30) Priorität: 25.04.2001 EP 01110261
(43) Veröffentlichungstag der Anmeldung: 21.01.2004
(73) Patentinhaber: WALDEMAR LINK GmbH & Co. KG, 22339 Hamburg (DE)
(72) Erfinder: KELLER, Arnold, 23863 Kayhude (DE)
(74) Vertreter: Glawe, Delfs, Moll
(86) Internationale Anmeldenummer: PCT/EP2002/004156
(87) Internationale Veröffentlichungsnummer: WO 2002/085257

(56) Entgegenhaltungen:
- EP-A- 0 278 184
- EP-A- 0 420 460

## Beschreibung

Die Erfindung bezieht sich auf Knieprothesen, die ein Scharnier zur Bestimmung der Beugebewegung enthalten. Es kann sich dabei um ein Scharnier handeln, das lediglich zur Stabilisierung dient (DE-C-31 19 841, EP-A-716 839, EP-A-791 343, EP-A-420 460) oder das die volle Last überträgt (GB-C-1 305 391, EP-A-278184, FR-C-2 161 588, US-A-3 772 709). Das Scharnier wird jeweils von einer Scharniergabel gebildet, die üblicherweise mit dem Oberschenkelknochen verbunden ist, und einem Scharniermittelstück, das mit dem Unterschenkelknochen verbunden ist. Die Schenkel der Scharniergabel sowie das Mittelstück enthalten fluchtende Bohrungen, die von einer Scharnierachse durchsetzt sind. Die femoralen und tibialen Komponenten der Prothese werden im allgemeinen zunächst gesondert implantiert und anschließend miteinander verbunden. In denjenigen Fällen, in denen lediglich der Scharnierbolzen die Komponenten miteinander verbindet, wird er nach der Implantation der Komponenten in die zugehörigen Lagerbohrungen von der Seite her eingeschoben. Dies macht eine entsprechende Einführungsöffnung in einer der Oberschenkelkondylen erforderlich, die den Knochen unerwünscht schwächt und zusätzlichen Aufwand während der Operation verlangt. Bei denjenigen Prothesen, die das Scharnier in einem stabilisierenden Teil enthalten, der mit der Tibiakomponente durch ein Rotationslager mit einem tibia-parallelen Zapfen verbunden ist, werden die Komponenten meist dadurch zusammengefügt, daß der zu der einen Komponente gehörige Zapfen in die Lagerbohrung der anderen Komponente eingeführt wird. Dies hat den Nachteil, daß der Bandapparat des Knies stark gedehnt werden muß.

Die Erfindung vermeidet diese Nachteile durch die im Anspruch 1 angegebenen Merkmale. Demgemäß ist vorgesehen, daß statt einer einheitlichen Scharnierachse zwei Achsstümpfe verwendet werden, die zwei Stellungen einnehmen können. In einer zur Montage verwendeten Stellung sind sie im wesentlichen vollständig innerhalb des Scharniermittelteils aufgenommen. Sie sind so weit in diesen Mittelteil zurückgezogen, daß dieser von der Seite her in die Scharniergabel eingesetzt werden kann. Danach werden die Achsstümpfe aus dem Mittelteil in die Stellung herausgeschoben, die sie im montierten Zustand innehaben und in der sie hinreichend weit in in den Gabelschenkeln enthaltene Lagerbohrungen vorragen.

Die Achsstümpfe benötigen keine große Stützlänge im Scharniermittelteil, weil der Abstand zwischen den Stirnflächen des Mittelteils und den Gabelschenkeln sehr gering ist und entsprechend gering auch das von den Achsstümpfen aufzunehmende Moment ist. Dennoch kann es zweckmäßig sein, wenn die Achsstümpfe Stützvorsprünge aufweisen, die an der Wandung der Scharnierbohrung des Mittelteils und/oder an dem jeweils anderen Achsstumpf stützend anliegen. Die Achsstümpfe mit den Vorsprüngen haben eine Länge, die größer ist als die halbe Länge der Scharnierbohrung im Mittelteil und vermögen daher etwa auftretende Momente sicher zu übertragen. In einer bevorzugten Ausführungsform wird der Stützvorsprung eines Achsstumpfs von einem Zapfen gebildet, der in dem als Zylinder ausgebildeten Stützvorsprung des anderen Achsstumpfs geführt ist. Es sind aber auch andere Stützausführungen denkbar. Beispielsweise kann jeder Achsstumpf am Umfang einen Kranz kammartig beabstandeter Vorsprünge tragen, die sich im Montagezustand über nahezu die gesamte Länge der Bohrung des Mittelteils zwischen den gegensinnig gerichteten Vorsprüngen des anderen Achsstumpfs erstrecken und sich jeweils am Umfang der Lagerbohrung des Mittelteils abstützen.

Um die Montage zu vereinfachen, können die Achsstümpfe unter der Spreizkraft einer Feder stehen, die sie in die montierte Stellung auseinander führt, sobald ihnen dies beim Erreichen der Lagerbohrungen in den Gabelschenkeln möglich ist. Beispielsweise wird der Scharniermittelteil bei Beginn der Montage zwischen Daumen und Zeigefinger so gehalten, daß die Achsstümpfe in den Mittelteil eingedrückt sind. Dieser wird dann in den Raum zwischen den Gabelschenkeln eingeschoben, so daß die Achsstümpfe nun durch ihre Anlage an der Stirnfläche der Gabelschenkel im Mittelteil zurückgehalten werden. Erst wenn die Lagerbohrungen erreicht sind, schnappen die Achsstümpfe unter der Wirkung der Spreizfeder auseinander in die montierte Stellung.

Damit aber der Operateur seine Aufmerksamkeit nicht auf das Festhalten der Achsstümpfe bei Beginn der Montage zu konzentrieren braucht, kann eine Halteeinrichtung vorgesehen sein.

Diese soll zumindest einen Teil der Achsstumpfstirnfläche zum Einfügen in die Scharniergabel frei lassen, damit die Halteeinrichtung gelöst werden kann, sobald der Scharniermittelteil mit den Achsstümpfen zumindest teilweise in die Scharniergabel eingesetzt ist.

Zur Demontage ist es notwendig, die Achsstümpfe aus den Lagerbohrungen der Scharniergabel wieder zurückzuziehen. Zu diesem Zweck ist in der Umfangsfläche des Mittelteils an einer in der Operation zugänglichen Stelle eine Öffnung vorgesehen, durch die geeignete Angriffsflächen der Achsstümpfe für ein Werkzeug zugänglich sind. Bei dieser Öffnung kann es sich um ein Langloch oder zwei je einem Achsstumpf zugeordnete Langlöcher handeln. Das Werkzeug kann auch zum Unterstützen des Spreizvorgangs eingesetzt werden, falls die Federkraft dazu nicht ausreichen sollte oder falls der Arzt sich vergewissern möchte, daß die Achsstümpfe ihre montierte Endstellung erreicht haben.

Es ist nicht erforderlich, die Achsstümpfe in der montierten Stellung zu sichern, da nicht mit Kräften gerechnet werden muß, durch die sie in die Montagestellung zurückgedrückt werden könnten. Wenn man sichergehen will, wird man jedoch eine solche Sicherungseinrichtung vorsehen.

Die Erfindung wird im folgenden näher unter Bezugnahme auf die zeichnung erläutert, die ein vorteilhaftes Ausführungsbeispiel veranschaulicht. Es zeigen:
- Fig. 1: eine Dorsalansicht der wesentlichen Teile der Prothese im gestreckten Zustand,
- Fig. 2: eine Ansicht der femoralen Komponente schräg von der Seite,
- Fig. 3: eine Ansicht der tibialen Komponente schräg von der Seite,
- Fig. 4: eine Ansicht dieser Teile während der Montage schräg von der Seite,
- Fig. 5: eine Schnittansicht des Scharniermittelteils mit den Achsstümpfen in Montagestellung,
- Fig. 6: eine Schnittansicht des Scharniers mit den Achsstümpfen in montierter Stellung,
- Fig. 7: eine Teilansicht des Mittelteils mit den durch eine Haltevorrichtung in der Montagestellung gehaltenen Achsstümpfen,
- Fig. 8: eine perspektivische Ansicht der Halteeinrichtung und
- Fig. 9: eine der Fig. 7 entsprechende Stellung mit durch ein Werkzeug gespreizten Achsstümpfen.

Die Prothese besteht aus einer tibialen Komponente 1, deren Dorn 2 in den Unterschenkelknochen einzusetzen ist, und einer femoralen Komponente 3, deren Dorn 4 in den Oberschenkelknochen einzusetzen ist. Die tibiale Komponente 1 endet oben in einer Platte 5, die ein Polyethylenplateau 6 trägt, dessen Oberseite eine Gleitfläche bildet für die Kufen 7 der femoralen Komponente. Über diese Teile wird die vertikale Last übertragen.

Zur Stabilisierung der Komponenten 1 und 3 ist eine Stabilisierungsanordnung vorgesehen, die ein Scharnier, das eine Beugebewegung zwischen den Komponenten 1 und 3 gestattet, und ein Rotationslager bildet. Die femorale Komponente 3 bildet in Form der kastenartig zueinander parallel angeordneten Platten 8 eine Scharniergabel mit Lagerbohrungen 9, die von hutförmigen Polyethylenlagerteilen 10 ausgekleidet sind, die eine hohlzylindrische Lagerfläche 11 bilden. Darin sind Achsteile 12, 13 gelagert, deren anderes Ende in dem Scharniermittelteil 14 angeordnet sind, das einstückig am oberen Ende eines Zapfens 15 angeordnet ist, der drehbar in einer Lagerbohrung 16 gelagert ist, die etwa parallel zu dem Dorn 2 in der Tibiakomponente 1 vorgesehen ist. Diese Stabilisierungsanordnung sorgt dafür, daß die Relativbewegung zwischen der Femur- und der Tibiakomponente ausschließlich als Beugebewegung um die Achse 19 der Achsstümpfe 12 oder als Rotationsbewegung um die durch die Bohrung 16 festgelegte Rotationsachse stattfinden kann.

Der Scharniermittelteil 14 sitzt nahezu spielfrei zwischen den Schenkeln 8 der Scharniergabel, wobei die Krempen 17 der Polyethylenteile 10 einen unerwünschten Metall/Metall-Kontakt vermeiden.

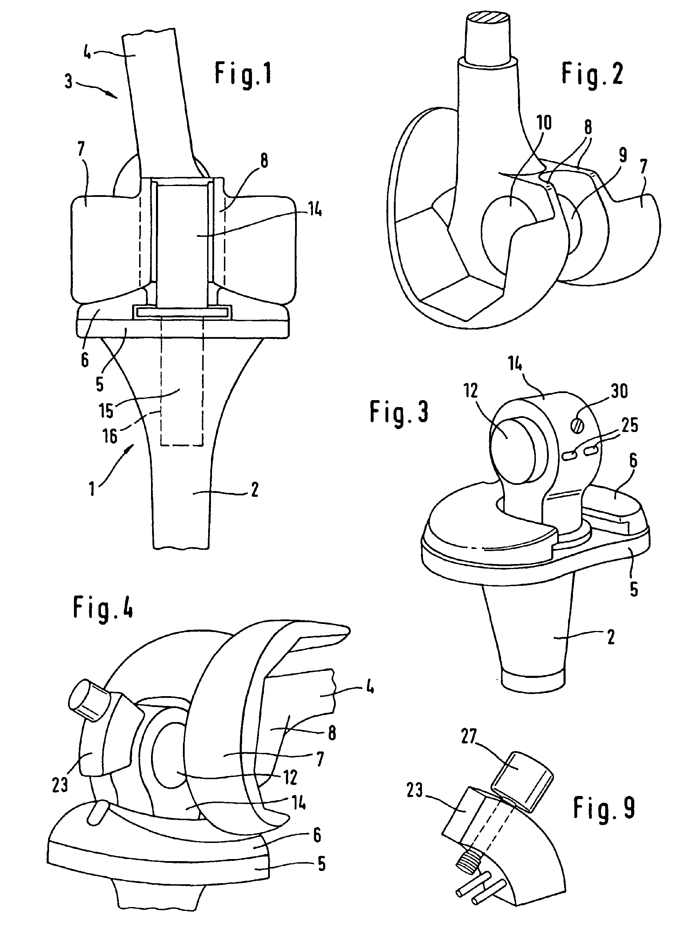

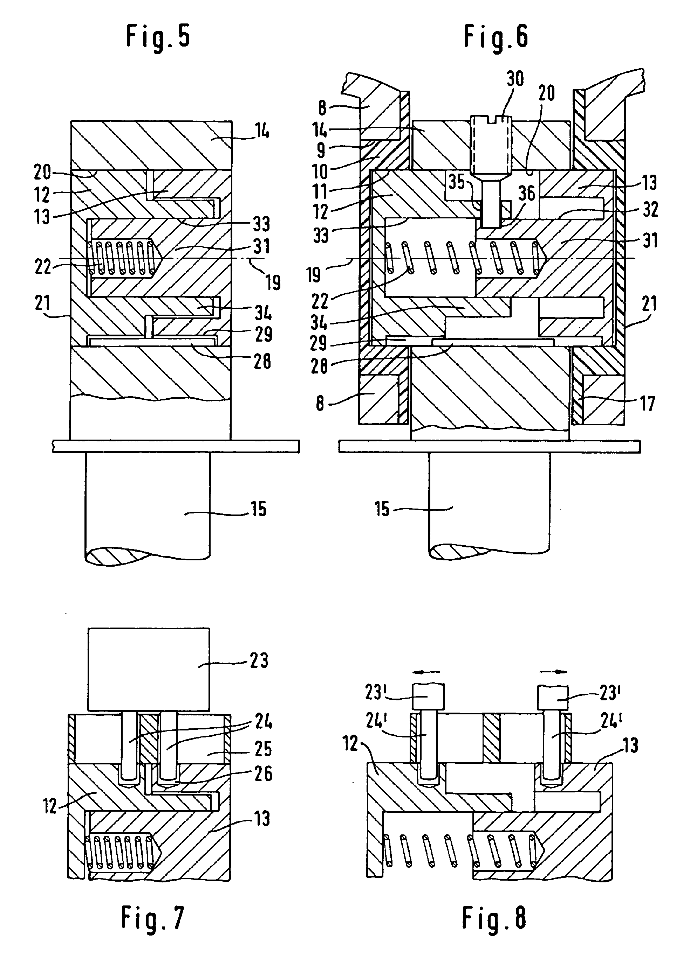

Wie Fig. 5 zeigt, können die Achsstümpfe 12, 13 in die Bohrung 20 des Scharniermittelteils 14 zurückgezogen bzw. eingedrückt werden, um die Montagestellung einzunehmen. Ihre Stirnflächen 21 fluchten dann etwa mit den Stirnflächen des Scharniermittelteils 14 und sind jedenfalls zusammen nicht breiter als der zur Montage zur Verfügung stehende Zwischenraum zwischen den Gabelschenkeln 8. Am Umfang ist die axiale Abmessung jedes Achsstumpfs 12, 13 etwa halb so groß wie die entsprechende Abmessung des Scharniermittelteils 14. Durch eine Druckfeder 22 werden sie aus dieser Montagestellung in die im montierten Zustand einzunehmende Stellung gedrängt. Damit dies nicht vorzeitig geschieht, werden sie gemäß Fig. 7 durch eine Halteeinrichtung 23 in der Montagestellung festgehalten. Diese Halteeinrichtung, die gesondert in Fig. 9 dargestellt ist, weist zwei in bestimmtem, unveränderlichem Abstand stehende Stifte 24 auf, die durch Langlöcher 25 im Scharniermittelteil 14 in Bohrungen 26 der Achsstümpfe 12, 13 greifen. Die Halteeinrichtung 23 kann in dieser Sicherungsstellung mittels einer Schraube 27 an dem Scharniermittelteil 14 temporär sicher gehalten werden.

Solange sich die Stabilisierungseinrichtung in diesem Montagezustand befindet, können die Tibiakomponente 1 und die Femurkomponente 3 zusammengefügt werden, wie Fig. 4 dies zeigt. Sobald sich der Scharniermittelteil 14 mit den Achsstümpfen 12, 13 zwischen den Gabelteilen 8 befindet, kann die Halteeinrichtung 23 gelöst und entfernt werden. Sobald die Komponenten diejenige Relativstellung erreicht haben, in der die Bohrungen in den Gabelschenkeln 8 und im Scharniermittelteil 14 miteinander fluchten, schnappen die Achsstümpfe 12, 13 unter der Wirkung der Feder 22 auseinander und in die Lagerbohrungen 11. Mittels eines Werkzeugs 23', von dem in Fig. 8 lediglich die Stifte 24' gezeigt sind, können die Achsstümpfe 12, 13 weiter gespreizt werden, falls sie nicht schon unter der Wirkung der Feder 22 ihre Endstellung erreicht haben sollten. Anschließend kann der Arzt die Achsstümpfe 12, 13 in der montierten Stellung mittels einer Schraube 30 sichern, die in der in Fig. 6 gezeigten Weise in Bohrungen 35, 36 von sTützvorsprüngen 31, 34 ein, die an den Achsstümpfen 12, 13 vorgesehen sind.

Damit die Bohrungen 26 durch die Langlöcher 25 hindurch stets erreichbar sind und nicht infolge Verdrehung der Achsstümpfe 12, 13 verschwinden, ist eine Verdrehsicherung vorgesehen, nämlich in der Bohrung 20 eine Längsrippe 28, die mit entsprechenden Nuten 29 der Achsstümpfe 12, 13 zusammenwirkt.

Wenn der Scharniermittelteil schmal ausgeführt wird, wie es erwünscht ist, wirken die Achsstümpfe 12, 13 nur über eine verhältnismäßig geringe axiale Länge mit der sie aufnehmenden Bohrung 20 des Scharniermittelstücks 14 zusammen. Obgleich sie nicht oder nur wenig durch Momente belastet werden, erscheint es zweckmäßig, für eine zusätzliche Abstützung zu sorgen. Dies geschieht im dargestellten Beispiel dadurch, daß der Achsstumpf 13 einen Stützzapfen 31 aufweist, dessen zylindrische Umfangsfläche 32 eng geführt ist in einer zylindrischen Bohrung 33 eines Stützvorsprungs 34 des Achsstumpfs 12. Diese Flächen bleiben auch im montierten Zustand in einem einander abstützenden und führenden Zustand.

Will man die Prothesenkomponenten wieder voneinander trennen, so zieht man mit dem in Fig. 8 angedeuteten Werkzeug die Achsstümpfe 12, 13 wieder zuammen. Der Scharniermittelteil kann dann aus der Scharniergabel 8 gelöst werden.

## Patentansprüche

1. Knieprothese mit einem Beugescharnier, das von einer Scharniergabel mit zwei einander gegenüberliegenden Schenkeln (8), einem in die Scharniergabel quer zu deren Beugeachse einzusetzenden Mittelteil (14) und einer Scharnierachse gebildet ist, die nach dem Einsetzen des Mittelteils (14) in die Scharniergabel durch Bewegung in ihrer Längsrichtung in fluchtenden Scharnierbohrungen (10, 20) der Scharniergabel und des Mittelteils (14) zu montieren ist, **dadurch gekennzeichnet, daß** die Scharnierachse von zwei Achsstümpfen (12, 13) gebildet ist, die in einer Montagestellung innerhalb des Mittelteils (14) angeordnet sind und in einer montierten Stellung teilweise daraus hervorragen.

2. Knieprothese nach Anspruch 1, **dadurch gekennzeichnet, daß** die Achsstümpfe (12, 13) Stützvorsprünge (31, 34) aufweisen, die an der Wandung der Scharnierbohrung (20) des Einsatzteils (14) und/oder an dem jeweils anderen Achsstumpf (13, 12) anliegen und sich über die halbe Länge der Scharnierbohrung (20) des Einsatzteils (14) hinaus erstrecken.

3. Knieprothese nach Anspruch 2, **dadurch gekennzeichnet, daß** der Stützvorsprung eines Achsstumpfs (13) von einem Zapfen (31) gebildet ist, der in dem als Zylinder ausgebildeten Stützvorsprung (34) des anderen Achsstumpfs (12) geführt ist.

4. Knieprothese nach einem der Ansprüche 1 bis 3, **dadurch gekennzeichnet, daß** die Achsstümpfe (12, 13) unter der Spreizkraft einer Feder (22) stehen.

5. Knieprothese nach Anspruch 4, **dadurch gekennzeichnet, daß** zum Halten der Achsstümpfe (12, 13) in der Montagestellung eine Halteeinrichtung (23) vorgesehen ist, die zumindest einen Teil der Achsstumpfstirnfläche (21) zum Einfügen zwischen die Gabelschenkel (8) frei läßt.

6. Knieprothese nach Anspruch 5, **dadurch gekennzeichnet, daß** die Achsstümpfe (12, 13) durch eine Öffnung (25) in der Umfangsfläche des Einsatzteils (14) für ein Spreiz-und/oder Zurückziehwerkzeug (23') zugänglich sind.

7. Knieprothese nach einem der Ansprüche 1 bis 6, **dadurch gekennzeichnet, daß** eine Einrichtung (30, 35, 36) zum Sichern der Achsstümpfe (12, 13) in der montierten Stellung vorgesehen ist.

## Claims

1. Knee prosthesis with a flexion hinge which is formed by a hinge fork with two branches (8) lying opposite each other, by a centre part (14) which is to be fitted into the hinge fork transversely with respect to the flexion axis of the latter, and by a hinge pin which, after the centre part (14) has been fitted into the hinge fork, is to be mounted, by movement in its longitudinal direction, in aligned hinge bores (10, 20) of the hinge fork and of the centre part (14), **characterized in that** the hinge pin is formed by two pin stumps (12, 13) which, in an assembly position, are arranged inside the centre part (14) and, in a mounted position, partially protrude from the latter.

2. Knee prosthesis according to Claim 1, **characterized in that** the pin stumps (12, 13) have support projections (31, 34) which bear on the wall of the hinge bore (20) of the insert part (14) and/or on the respective other pin stump (13, 12) and extend out over half the length of the hinge bore (20) of the insert part (14).

3. Knee prosthesis according to Claim 2, **characterized in that** the support projection of one pin stump (13) is formed by a journal (31) which is guided in the support projection (34), designed as a cylinder, of the other pin stump (12).

4. Knee prosthesis according to one of Claims 1 to 3, **characterized in that** the pin stumps (12, 13) are subject to the spreading force of a spring (22).

5. Knee prosthesis according to Claim 4, **characterized in that**, in order to hold the pin stumps (12, 13) in the assembly position, a retaining device (23) is provided which leaves free at least part of the end face (21) of the pin stump for insertion between the fork branches (8).

6. Knee prosthesis according to Claim 5, **characterized in that** the pin stumps (12, 13) can be accessed by an instrument (23') for spreading them and/or drawing them back together, which instrument is introduced through an opening (25) in the circumferential surface of the insert part (14).

7. Knee prosthesis according to one of Claims 1 to 6, **characterized in that** a means (30, 35, 36) is provided for securing the pin stumps (12, 13) in the mounted position.

## Revendications

1. Prothèse du genou pourvue d'une charnière de flexion, qui est formée par une fourche de charnière comportant deux branches (8) opposées l'une à l'autre, une pièce centrale (14) à introduire dans la fourche de charnière transversalement à son axe de flexion et un axe de charnière, qui doit être monté dans des alésages de charnière alignés (10, 20) de la fourche de charnière et de la pièce centrale (14) par un mouvement dans sa direction longitudinale après l'insertion de la pièce centrale (14) dans la fourche de charnière, **caractérisée en ce que** l'axe de charnière est formé par deux bouts d'axe (12, 13) qui, dans une position de montage, sont disposés à l'intérieur de la pièce centrale (14) et, dans une position montée, sortent partiellement de celle-ci.

2. Prothèse du genou selon la revendication 1, **caractérisée en ce que** les bouts d'axe (12, 13) présentent des saillies d'appui (31, 34), qui reposent sur la paroi de l'alésage de charnière (20) de la pièce d'insertion (14) et/ou respectivement sur l'autre bout d'axe (13, 12) et s'étendent sur la moitié de la longueur de l'alésage de charnière (20) de la pièce d'insertion (14).

3. Prothèse de genou selon la revendication 2, **caractérisée en ce que** la saillie d'appui d'un bout d'axe (13) est formée par un tourillon (31), qui est guidé dans la saillie d'appui (34) de l'autre bout d'axe (12) de forme cylindrique.

4. Prothèse du genou selon l'une quelconque des revendications 1 à 3, **caractérisée en ce que** les bouts d'axe (12, 13) sont soumis à la force d'expansion d'un ressort (22).

5. Prothèse du genou selon la revendication 4, **caractérisée en ce qu'**il est prévu, pour maintenir les bouts d'axe (12, 13) dans la position de montage, un dispositif de retenue (23), qui laisse libre au moins une partie de la face frontale (21) du bout d'axe en vue de l'insertion entre les branches (8) de la fourche.

6. Prothèse du genou selon la revendication 5, **caractérisée en ce que** les bouts d'axe (12, 13) sont accessibles pour un outil d'expansion et/ou de retrait (23') par une ouverture (25) dans la surface périphérique de la pièce d'insertion (14).

7. Prothèse du genou selon l'une quelconque des revendications 1 à 6, **caractérisée en ce qu'**il est prévu un dispositif (30, 35, 36) pour bloquer les bouts d'axe (12, 13) dans la position montée.
